# EUROPEAN PATENT SPECIFICATION

(11) **EP 1 526 106 B1**
(45) Date of publication and mention of the grant of the patent: **25.05.2005**
(21) Application number: 03256683.8
(22) Date of filing: 23.10.2003
(51) Int. Cl.: B66B 31/02, B66B 31/00, A61L 2/24, A61L 2/26

(54) **Rotary belt sterilizer**
Rotierender Riemen Sterilisator
Stérilisateur de courroie rotative

(43) Date of publication of application: 27.04.2005
(73) Proprietor: Arai Works Co. Ltd., Iwatsuki-shi, Saitama 339-0072 (JP)
(72) Inventor: Masuda, Minoru, Chiba-shi Chiba 267-0066 (JP); Suzuki, Yoshio, Koshigaya-shi Saitama 343-0834 (JP)
(74) Representative: Townsend, Victoria Jayne

(56) References cited:
- US-B1- 6 321 900
- PATENT ABSTRACTS OF JAPAN vol. 1998, no. 03, 27 February 1998 (1998-02-27) & JP 09 290989 A (MASUDA MINORU), 11 November 1997 (1997-11-11)
- PATENT ABSTRACTS OF JAPAN vol. 1997, no. 12, 25 December 1997 (1997-12-25) & JP 09 208173 A (HITACHI BUILDING SYST CO LTD), 12 August 1997 (1997-08-12)
- PATENT ABSTRACTS OF JAPAN vol. 2002, no. 11, 6 November 2002 (2002-11-06) & JP 2002 205890 A (MASUDA MINORU), 23 July 2002 (2002-07-23)

## Description

### BACKGROUND OF THE INVENTION

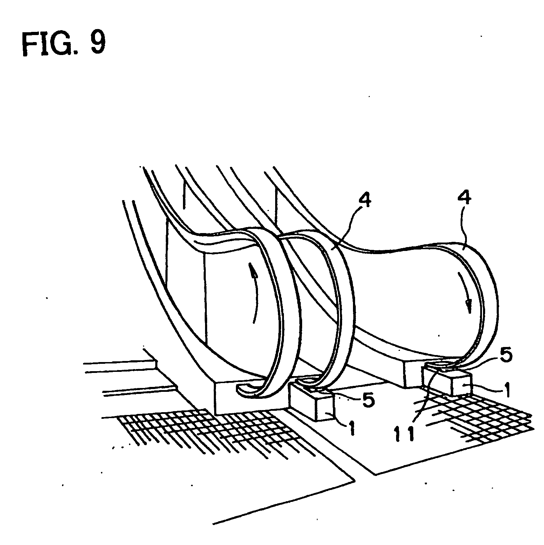

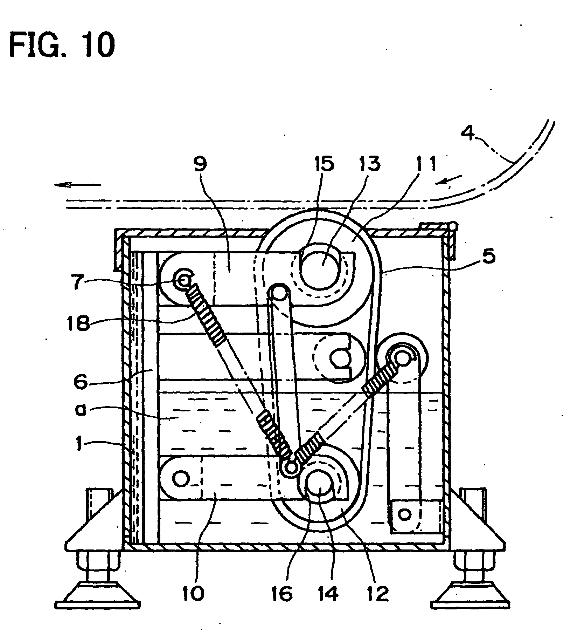

Japanese Patent No. 3004588 discloses a conventional rotary belt sterilizer, which is also invented by the present Inventors. As shown in Figs. 9 and 10, the rotary belt sterilizer comprises a sterilizing rotary belt 5 that press-contacts a sterilized rotary belt 4 resiliently by the aid of a resilient mechanism and passes through a sterilizing solution-a contained in a casing 1. It also comprises a throttle mechanism arranged to appropriately adjust the amount of the sterilizing solution soaked in the sterilizing rotary belt 5. The resilient mechanism includes a pair of upper and lower movable links 9 and 10, of which end is each attached pivotally on a stationary link 6 in a link mechanism. By bearings 15 and 16 located at free ends of the movable links 9 and 10, rotary shafts 13, 14 of drums 11, 12 for the sterilizing rotary belt 5 are supported pivotally. A tensible spring 18 is detachably provided between a pivot 7 of the upper movable link 9 and the free end of the lower movable link 10.

In the above rotary belt sterilizer, however, the sterilized rotary belt 4 normally press-contacts the sterilizing rotary belt 5. In addition, a part of the sterilizing rotary belt 5 is always immersed into the sterilizing solution-a. Therefore, the sterilized rotary belt 4 is kept soaked with the sterilizing solution a until it disappears. Thus, for example, after the sterilized rotary belt 4 shaped in endless annular rotates one turn, further sterilization may not be required. Even though, as long as the sterilizing solution remains, sterilization is continued. Accordingly, even if a necessary amount of the sterilizing solution is calculated previously and the sterilizing solution is stored in the casing, the sterilizing solution may be consumed uselessly. Further, after complete consumption of the sterilizing solution, the next sterilizing solution should be supplied manually at an appropriate time with a troublesome management. In addition, as the sterilized rotary belt 4 normally press-contacts the sterilizing rotary belt 5, the sterilizing rotary belt 5 suffers distortions and abrasions. Therefore, a long-term use requires expensive maintenance costs such as a cost for replacement of the sterilizing rotary belt 5 and a labor cost for the replacement. In addition, the sterilizing rotary belt 5 press-contacts the sterilized rotary belt 4 during halts on running of the sterilized rotary belt 4. Accordingly, when the sterilized rotary belt 4 is resumed to run, stains caused from contact with the sterilizing rotary belt 5 and solidification of the sterilizing solution may stay on the surface of the sterilized rotary belt 4. Such stains cause persons to hold a sense of contamination, which should be removed manually with necessary labors.

To solve such the problems, the present invention has an object to separate an applicator roller for the sterilizing solution from a sterilized rotary belt during halts of the rotary belt and contact the applicator roller with the rotary belt during running of the rotary belt. This is effective to prevent stains, caused from contact with the applicator roller and solidification of a cleaning solution, from staying on the rotary belt. In addition, distortions and abrasions of the rotary belt and the applicator roller can be reduced as low as possible. Further, only a sufficient amount of the sterilizing solution is supplied to the rotary belt to eliminate useless consumption of the sterilizing solution. A timer function is provided in the device itself to supply the sterilizing solution periodically and automatically to save costs for maintenance and management of the device.

### SUMMARY OF THE INVENTION

To achieve the above object, the present invention provides a rotary belt sterilizer comprising: a drive roller arranged to rotate in synchronization with running of a sterilized rotary belt; an applicator roller for the sterilizing solution configured to apply a sterilizing solution to said sterilized rotary belt; a switching device for the applicator roller arranged to contact said applicator roller with said sterilized rotary belt during rotations of said drive roller and separate said applicator roller from said sterilized rotary belt during halts of said drive roller; a supply tray for the sterilizing solution arranged to supply said sterilizing solution to said applicator roller; and a sterilizing solution supplier arranged to supply said sterilizing solution to said supply tray from a storage tank for the sterilizing solution per pre-determined rotations of said drive roller. Said drive roller, said applicator roller, said switching device, said supply tray and said sterilizing solution supplier are provided in a casing. Preferably, the drive roller is one of a pair of left and right drive rollers normally contacted with said rotary belt. Thus, it is possible to automatically control actuation of each mechanism based on rotations and halts of the drive roller or the number of rotations thereof.

Preferably, the switching device includes: a gear provided on a deceleration rotary shaft arranged to rotate/halt in response to rotations/halts of one of said drive rollers; an eccentric gear arranged to detachably mate with said gear; a lifting rod for the applicator roller arranged to lift said applicator roller up and down in response to rotations/halts of said eccentric gear; a flywheel arranged to rotate/halt in response to rotations/halts of the other of said drive rollers; and an engaging unit for the lifting rod arranged to engage with/disengage from said lifting rod in response to rotations/halts of said flywheel when said applicator roller is lifted up. Such the switching device enables the applicator roller to contact with and separate from the rotary belt in response to running and halt of the rotary belt. Thus, distortions and abrasions of the rotary belt and the applicator roller as well as stains due to the cleaning solution staying on the rotary belt can be reduced as low as possible.

Preferably, the engaging unit includes: a weight movable toward the perimeter in response to a centrifugal force caused by rotations of said flywheel; an actuation pin arranged axially movable on the axial center of a rotary shaft of said flywheel; an axial movement converter mechanism configured to move said actuation pin axially in response to movement of said weight toward the perimeter; a vertical movement converter mechanism configured to convert axial movement of said actuation pin into vertical movement; and an engagement hook configured to complete preparation of engagement with said lifting rod when said vertical movement converter mechanism provides down pressure and disengage from said lifting rod when said vertical movement converter mechanism releases pressure. The use of such the lifting rod engaging unit enables the applicator roller to contact with and separate from the rotary belt automatically. As a result, it is possible to achieve effective use of the sterilizing solution and easy maintenance/management of the rotary belt sterilizer.

The axial movement converter mechanism includes: a pivotal lever having a lever end and configured to pivot along the axis of said flywheel in response to movement of said weight toward the perimeter to press said lever end against an end of said actuation rod; and a spring means configured to normally resiliently press or pull said actuation rod to release pressure applied on said engagement hook. The use of such the mechanism enables rotations of the drive roller to be converted into linear motion easily and reliably. The sterilizing solution supplier includes: a deceleration mechanism containing a worm provided on said deceleration rotary shaft and a worm wheel having a flat cum mated with said worm; a supply pump for the sterilizing solution being actuated from a guide pin that impinges on said flat cum in said deceleration mechanism; and a storage tank for the sterilizing solution arranged in communication with said supply pump. The use of such the sterilizing solution supplier enables, in consideration of a deceleration rate of the decelerator, a necessary amount of the sterilizing solution to be supplied from the storage tank to the supply tray periodically and automatically. This is effective to reduce a loss in the sterilizing solution as low as possible.

Preferably, the applicator roller contacts a supply roller for the sterilizing solution that is partly immersed into said sterilizing solution in said supply tray and arranged rotatable therein. A throttle roller is provided with said supply roller. The supply pump has an inlet and an outlet each provided with a unidirectional valve in a flow direction of said sterilizing solution. With this arrangement, the applicator roller is provided with an improved durability and kept at an average humidity. In addition, the sterilizing solution is prevented from reverse flowing, which otherwise causes the sterilizing solution residing in the supply pipe and the casing to leak to external possibly during replacement of the supply pump. The storage tank is detachably attached to said casing, and wherein said storage tank and said casing each have a unidirectional valve at an aperture thereof for attachment to another. The unidirectional valve being opened on attachment and closed on detachment. This configuration is preferable because it is rich in workability for attachment and detachment of the storage tank to and from the casing when the sterilizing solution is supplied to the storage tank.

### BRIEF DESCRIPTION OF THE DRAWINGS

The present invention will be more fully understood from the following detailed description with reference to the accompanying drawings, in which:
Fig. 1 is an outlined illustrative view showing the entire of an embodiment of a rotary belt sterilizer according to the present invention;
Fig. 2 is a perspective view showing a partly cutoff flywheel portion in Fig. 1;
Fig. 3 is a perspective view of the entire except for a casing in Fig. 1 seen from the front right direction;
Fig. 4 is a detailed illustrative view of a sterilizing solution supplier in Fig. 1;
Fig. 5 is a partly enlarged illustrative view of a switching device for the applicator roller in Fig. 1;
Fig. 6 is a perspective view of the entire except for the casing in Fig. 1 seen from the front left direction;
Fig. 7 is a plan view of the entire except for the casing in Fig. 1;
Fig. 8 is an associated illustrative view of a worm wheel, a piston drive arm, and a supply pump for the sterilizing solution ;
Fig. 9 is an illustrative view showing an exemplary use of a conventional rotary belt sterilizer; and
Fig. 10 is a cross-sectional view of the major part of the conventional rotary belt sterilizer.

### DETAILED DESCRIPTION OF THE PREFERRED EMBODIMENTS

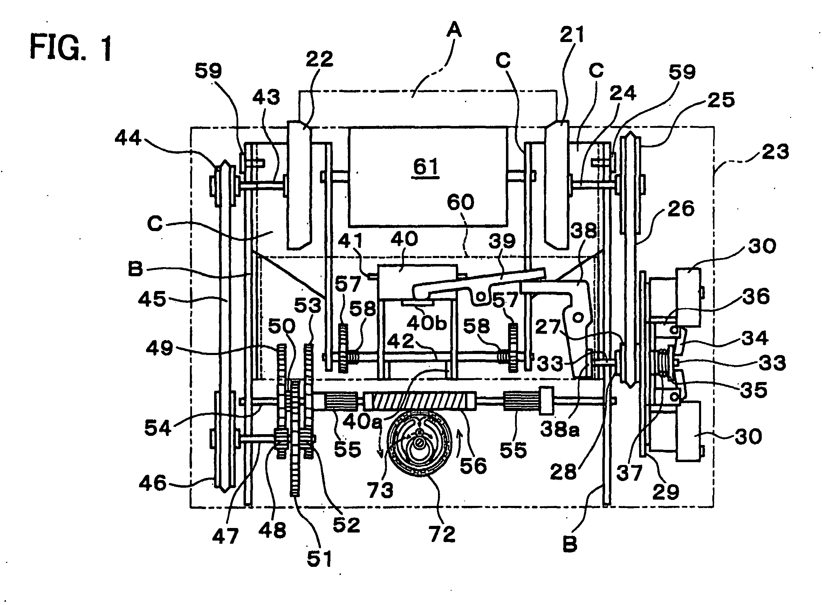

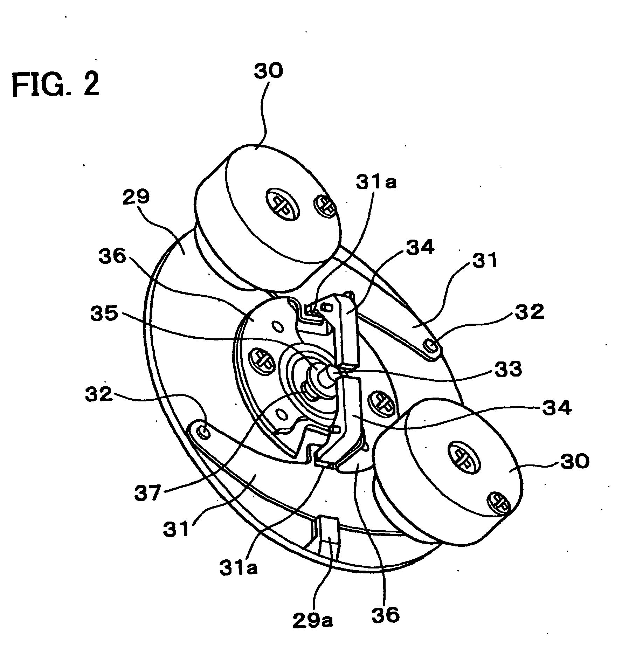

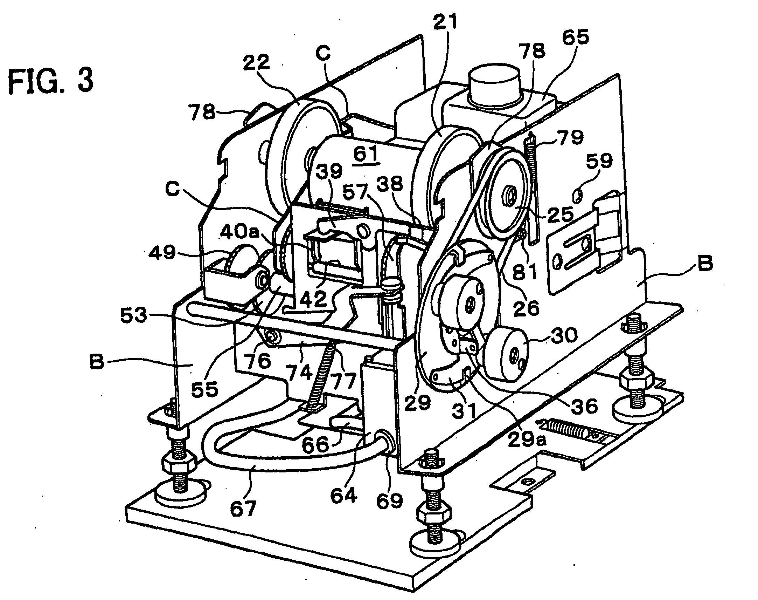

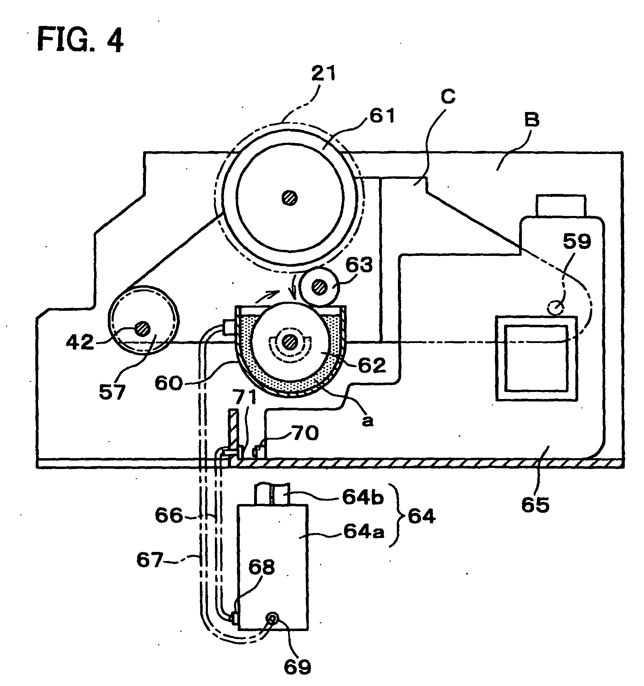

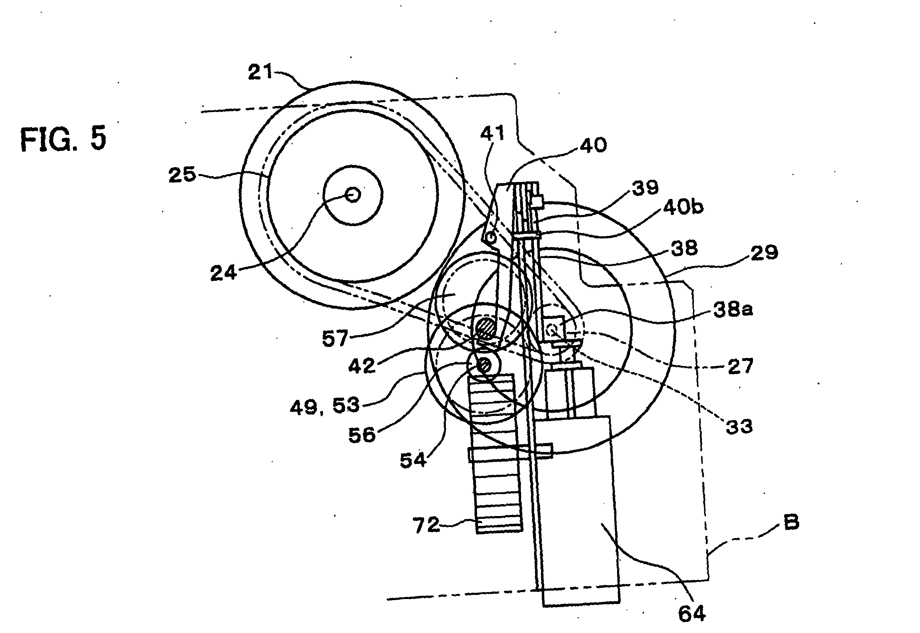

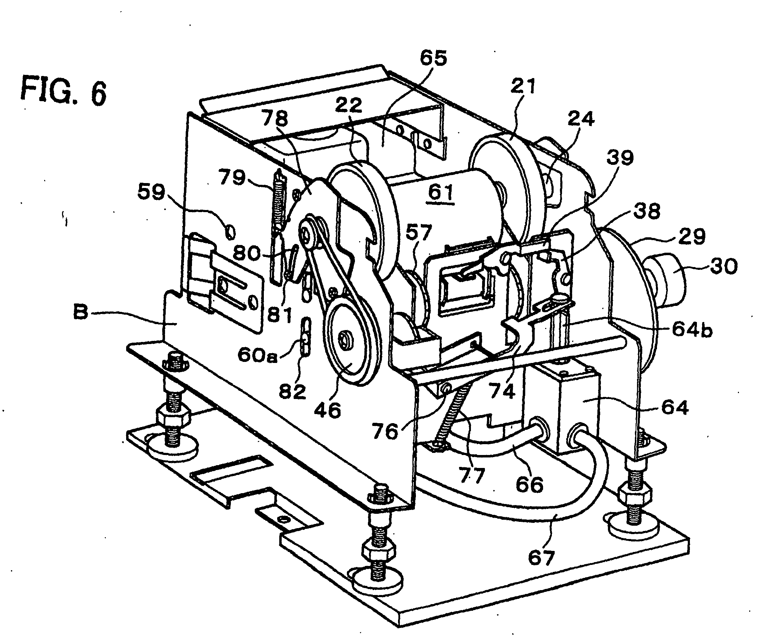

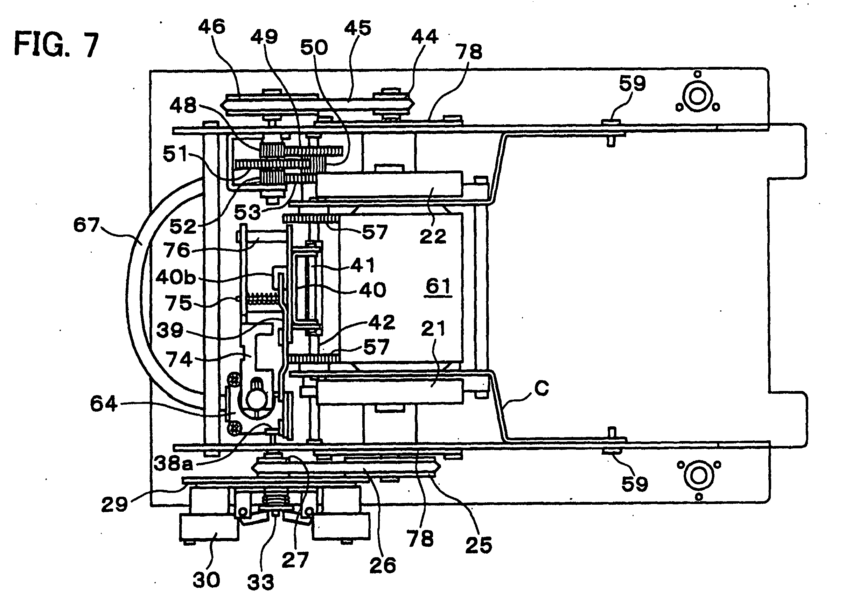

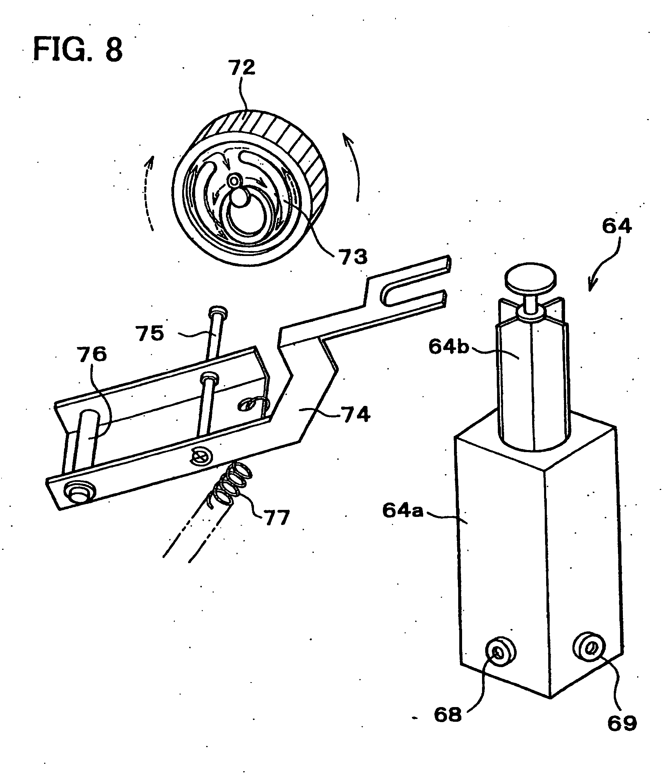

Embodiments of the rotary belt sterilizer according to the present invention will be described with reference to the drawings. Fig. 1 is an outlined illustrative view showing the entire of the rotary belt sterilizer according to the present invention. Fig. 2 is a perspective view showing a partly cutoff flywheel portion. Fig. 3 is a perspective view of the entire except for a casing seen from the front right direction. Fig. 4 is a detailed illustrative view of a sterilizing solution supplier. Fig. 5 is a partly enlarged illustrative view of a switching device for an applicator roller. Fig. 6 is a perspective view of the entire except for the casing seen from the front left direction. Fig. 7 is a plan view of the entire except for the casing. Fig. 8 is an associated illustrative view of a worm wheel, a piston drive arm, and a supply pump for the sterilizing solution.

In Fig. 1, the reference numerals 21 and 22 denote drive rollers, which are pivotally and rotatably attached to frames B in a casing 23 and press-contacted with a sterilized rotary belt A. One drive roller 21 is coupled to a flywheel 29 that is fixed to a rotary shaft 28 through a V-pulley 25 fixed to a rotary shaft 24 that is pivotally and rotatably attached to the frame B; a V-belt 26; and an acceleration V-pulley 27 fixed to a rotary shaft 28 that is pivotally attached to the frame B. As shown in Fig. 2, guide members 31 are provided on both sides of the flywheel 29 in the radial direction through the center thereof. The guide member 31 has one end pivotally supported on the flywheel 29 by a pivotal axis 32 and the other free end provided with a weight 30 attached thereto. The guide member 31 is employed to guide the weight 30 to move toward the perimeter of the flywheel 29 in response to a centrifugal force caused by rotations of the flywheel 29.

An inverted L-shaped actuation hook 34 is provided at an inner central location on each of the guide members 31. When the weight 30 moves toward the perimeter of the flywheel 29 about the pivotal axis 32 in response to the centrifugal force caused by rotations of the flywheel 29, the actuation hook 34 inhibits the flywheel 29 to rotate about the rotary shaft 28. At the same time, the actuation hook 34 presses a flange 35 of an axially movable actuation pin 33 to move the actuation pin 33 axially toward the center of the frame B. The actuation hook 34 has an inverted L-shaped corner pivotally supported by a pair of brackets 36, 36; the lower end fitted in a bore 31a formed through the guide member 31; and the upper end located as possible to contact with/separate from the flange 35 formed around the actuation pin 33.

Interposed between the flange 35 and the flywheel 29 is a spring 37 to restore the actuation pin 33 to the original location when the centrifugal force applied to the weight 30 is lost due to halts of the flywheel 29 and the weight 30 can not move toward the center of the flywheel 29 by its own weight. In the figure, the reference numeral 29a denotes a stopper for restricting the movable range of the guide member 31 for the weight 30 provided on the flywheel 29.

The tip of the actuation pin 33 located internally from the frame B is coupled to a transfer member 38 and a pivotal lever 39, which convert axial movement of the actuation pin 33 into vertical movement.

In a word, the tip of the actuation pin 33 contacts with a contacted portion 38a for the actuation pin formed on the lower end of the inverted L-shaped transfer member 38 that has the central portion pivotally attached to the frame B. The upper end of the transfer member 38 contacts with the lower side of one end of the pivotal lever 39 that has the central portion pivotally attached to the frame B. The other end of the pivotal lever 39 contacts with an engaging hook actuation member 40 that can freely engage with/disengage from a lifting rod 42 for the applicator roller as described later.

The engaging hook actuation member 40, having an engaging hook 40a at the lower end thereof, is attached to a front plate of the frame B such that the lower portion pivots to and from about an axis 41 at the upper end. The engaging hook actuation member 40 has an contacted portion 40b for contacting with the pivotal lever 39. When the pivotal lever 39 presses the contacted portion 40b, the lower end thereof pivots toward the inside of the frame B to prepare the engaging hook 40a so as to tilt to the lifting rod 42 and engage with it. When the lifting rod 42 is lifted up, it engages with the engaging hook 42a. When the pivotal lever 39 releases pressure, the engaging hook 40a is separated from the lifting rod 42. This operation is achieved by empty weights of later-described movable frames C and the lifting rod 42 attached thereto, and a restorative force caused when the lower portion of the engaging hook actuation member 40 restores to the original position about the axis 41 by its own weight. Thus, the engaging hook 40a is arranged to freely contact with and separate from the lifting rod 42.

As the engaging hook 40a, a substantially triangular hook with a rear end protruded backward is employed. When the lifting rod 42 elevates and engages with the engaging hook 42a, the lifting rod 42 elevates along the lower surface of the engaging hook 42a. Then, when the lifting rod 42 reaches a location above the engaging hook 42a, the engaging hook actuation member 40 pivots backward and engages with the lifting rod 42.

The other drive roller 22 is coupled through a rotary shaft 43 that is pivotally attached to a later-described drive roller arm 78; a V-pulley 44 fixed to the rotary shaft 43; a V-belt 45; a deceleration V-pulley 46 rotatably attached to the frame B; a rotary shaft 47 thereof; and a group of deceleration gears 48-53 to a deceleration rotary shaft 54 to rotate it in a deceleration mode.

In the group of deceleration gears, the gear 48 is fixed on the rotary shaft 47. The larger diameter gear 49 and the smaller diameter gear 50, mated with the gear 48, are rotatably attached to the deceleration rotary shaft 54. The larger diameter gear 51 and the smaller diameter gear 52, mated with the smaller diameter gear 50, are rotatably attached to the rotary shaft 47. The larger diameter gear 53 mated with the smaller diameter gear 52 is fixed to the deceleration rotary shaft 54. The group of the deceleration gears is thus configured.

A pair of left and right cylindrical gears 55, 55 are attached to the deceleration rotary shaft 54. A worm gear 56 is attached between both cylindrical gears 55, 55. The cylindrical gears 55, 55 can mate with a pair of eccentric gears 57, 57 that are rotatably attached to the lifting rod 42, as possible to contact with/ separate from it.

Provided between the lifting rod 42 and the eccentric gears 57, 57 are springs 58, 58 to lift the longer diameter portions of the eccentric gears 57, 57 upward when the lifting rod 42 falls down and the shorter diameter portions of the eccentric gears 57, 57 mate with the cylindrical gears 55, 55, and then the eccentric gears 57, 57 rotate from the longer diameter portions to the shorter diameter portions in response to rotations of the cylindrical gears 55, 55.

As shown in Fig. 4, the lifting rod 42 is supported rotatably on tips of a pair of left and right movable frames C, C. The movable frames C, C have rear ends pivotally attached to the frames B through shafts 59, 59. The tips of the movable frames C, C, or the portions applied to attach the lifting rod 42, are pivotally attached to the frames B as free ends. Beneath the rear of the lifting rod 42 between the movable frames C, C, a supply tray 60 for the sterilizing solution is resiliently suspended by the aid of springs, not shown, to freely lift up and down. Rotatably attached above the supply tray 60 is an applicator roller 61 for the sterilizing solution that can freely engage with/disengage from the sterilized rotary belt A. Located inside the supply tray 60 is a freely rotatable supply roller 62 for the sterilizing solution that is immersed into the sterilizing solution-a. Rotatably interposed between the movable frames C, C at a location suitable for contacting both the supply roller 62 and the applicator roller 61 is a throttle roller 63 that is arranged to adjust the amount of the sterilizing solution a soaked in the applicator roller 61.

The supply tray 60, the applicator roller 61, the supply roller 62 and the throttle roller 63 are integrally supported between the movable frames C, C resiliently by the aid of springs (not shown). They are thus configured to freely lift up and down relative to the movable frames C, C to absorb contact vibrations between the sterilized rotary belt A and the applicator roller 61 on running of the sterilized rotary belt A. In the figure, the reference numeral 60a denotes a projecting guide pin provided on both sides of the supply tray 60, and 82 denotes a lifting range restriction hole formed through the frame B.

The supply roller 60 is linked through flexible pipes 66, 67 to a storage tank 65 for the sterilizing solution through a supply pump 64 for the sterilizing solution consisting of a cylinder 64a and a piston 64b. The supply pump 64 has an inlet 68 provided with a unidirectional valve (not shown) that opens/closes in response to negative/positive pressure inside the pump, and an outlet 69 provided with a unidirectional valve (not shown) that opens/closes in response to positive/negative pressure inside the pump. The storage tank 65 has a supply aperture 70 for the sterilizing solution and a tank attachment aperture 71 attached to the frame B, to which the supply aperture 70 is detachably attached. These apertures are provided with switching valves (not shown) that open/close when both are coupled to/separated from each other.

The worm gear 56 attached to the deceleration rotary shaft 54 is mated with a worm wheel 72 provided with a flat cum 73 on the inner surface as shown in Fig. 8. Provided on the flat cum 73 is a guide pin 75 in the piston drive arm 74 that actuates the piston 64a in the supply pump 64. A shaft 76 is employed to pivotally attach the rear end of the piston drive arm 74 to the front plate of the frame B to allow the front end of the arm to freely lift up and down. The front end is coupled to the upper portion of the piston 64b in the supply pump 64. Fixed to the central portion of the arm 74 is the guide pin 75 that contacts with the flat cum 73 on the worm wheel 72. The piston drive arm 74 is provided with a tensioning spring 77 to normally pull the front end of the arm resiliently downward. The flat cum 73, the guide pin 75 and the spring 77 are thus configured to lift up and down the front end of the piston drive arm 74.

The drive rollers 21, 22 and the V-pulleys 25, 44 are

attached to the frames B, as shown in Figs. 6 and 7, through a resilient mechanism. This mechanism is configured to press-contact the drive rollers 21, 22 to the rotary belt A, and to absorb contact vibrations between the belt A and the drive rollers 21, 22 on running of the belt A. In a word, the drive rollers 21, 22 and the V-pulleys 25, 44 are attached to pivotable free ends of drive roller arms 78 that have one ends pivotally attached to the frames B and the other ends formed as the above free ends. Formed through the free ends of the drive roller arms 78 are pivotal range restrictive holes 80 that restrict pivotal ranges of the drive roller arms 78. Fitted in the pivotal range restrictive holes 80 are pins 81 that are fixed on the frames B. Provided at the free ends of the drive roller arms 78 are tensioning springs 79 that resiliently pull the drive roller arms 78 upward within the pivotal range restrictive holes 80.

An exemplary use and operation of the rotary belt sterilizer according to the embodiment will be described when the cleaner is employed at a side for drawing out a rotary belt in an escalator.

As same as the prior art shown in Fig. 9, the applicator roller 61 is located on the side for drawing out the endless annular rotary belt in the escalator and the storage tank 65 is located in front, so as to press-contact the drive rollers 21, 22 with the rotary belt A. When the escalator is activated to start running of the rotary belt A, the drive rollers 21, 22 are rotated by a frictional force with the rotary belt A. The rotation of the drive roller 21 is transmitted through the rotary shaft 24, the V-pulley 25, the V-belt 26, the acceleration V-pulley 27 and the rotary shaft 28 to the flywheel 29. The rotation of the flywheel 29 causes a centrifugal force, which moves the weight 30 toward the perimeter of the flywheel 29. The movement of the weight 30 toward the perimeter axially moves the actuation pin 33 internally from the frame B through the guide member 31, the actuation hook 34 and the flange 35. This axial movement is transmitted to the actuation member 40 through the transfer member 38 and the pivotal lever 39 to pivot the engaging hook 40a on the lower portion of the actuation member 40 toward the lifting rod 42.

The rotation of the drive roller 22 is transmitted through the rotary shaft 43, the V-pulley 44, the V-belt 45, the deceleration V-pulley 46, and the rotary shaft 47 to the group of deceleration gears 48-53 to rotate the deceleration rotary shaft 54, the cylindrical gear 55 and the worm gear 56 to rotate them in the deceleration mode. The cylindrical gear 55 is mated with the shorter diameter portion of the eccentric gear 57 before starting the sterilizer. On the central axis of the flat cum 73 in the worm wheel 72 mated with the worm gear 56, the guide pin 75 of the piston drive arm 74 is located. Therefore, when the deceleration rotary shaft 54 starts to rotate, the eccentric gear 57 and the worm wheel 72 also start to rotate at a pre-determined rate. When the eccentric gear 57 rotates to the longer diameter portion, the lifting rod 42 elevates up to the upper limit and engages with the engaging hook 40a of the actuation member 40 simultaneously. When the eccentric gear 57 and the cylindrical gear 55 further rotate after the lifting rod 42 engages with the engaging hook 40a, the eccentric gear 57 is released from mating with the cylindrical gear 55. As a result, the spring 58 lifts up the longer diameter portion of the eccentric gear 57.

When the lifting rod 42 elevates as shown in Fig. 4, the free end of the frame C is also lifted up about the shaft 59 simultaneously. In this case, the supply tray 60, the applicator roller 61, the supply roller 62 and the throttle roller 63 resiliently held on the free end are integrally lifted up. At the same time, the applicator roller 61 press-contacts the rotary belt A. After the applicator roller 61 press-contacts the rotary belt A, a time period determined from the gear ratio between the worm gear 56 and the worm wheel 72 elapses. That is, the guide pin 75 of the piston drive arm 74 guided by the flat cum 73 on the worm wheel 72 moves from the shortest diameter portion to the longest diameter portion of the flat cum 73. During this movement, the drive arm 74 lifts up the piston 64a in the supply pump 64 to absorb the sterilizing solution-a from the supply tank 65 into the supply pump 64. When the guide pin 75 on the drive arm 74 reaches the shortest diameter portion from the longest diameter portion on the axis of the flat cum 73, the guide pin 75 is pulled toward the rotary shaft of the flat cum 73 resiliently by the spring 77. Then, the drive arm 74 lifts down the piston 64b in the supply pump 64. As a result, the sterilizing solution-a absorbed in the cylinder 64a is supplied from the outlet 69 through the flexible pipe 67 to the supply tray 60.

The sterilizing solution-a supplied to the supply tray 60 is adjusted in attachment amount using the supply roller 62 and the throttle roller 63, then applied to the applicator roller 61, and finally applied to the rotary belt A. The sterilizing solution-a may be applied to the rotary belt A at an interval of, for example, two hours. To achieve this interval, it is required to adjust a deceleration ratio associated with the deceleration pulley 46, the group of deceleration gears 48-53, the worm gear 56 and the worm wheel 72. In this case, a combination of the rotational rate and the number of rotations of the drive roller 22 is employed to rotate the worm wheel 72 a half turn every two hours. This adjustment enables a certain amount of the sterilizing solution-a to be supplied to the supply tray 60 every two hours and the sterilizing solution-a is applied to the rotary belt A.

After the sterilizing solution-a in the supply tank 65 is consumed, the sterilizing solution-a is additionally supplied to the supply tank 65. The supply tank 65 is removed from the frame B and the cap thereof is opened before the sterilizing solution-a is supplied. In this case, the supply aperture 70 of the supply tank 65 is provided with a switching valve to close the supply aperture 70 when the connection with the tank attachment aperture 71 is released. Therefore, the sterilizing solution-a is prevented from leaking out of the supply tank 65.

When a halt is made on operation of the escalator to stop running of the rotary belt A, the drive rollers 21, 22 also stop rotating. When one drive roller 21 stops rotating, the flywheel 29 provided in communication with the roller 21 also stops. As a result, a centrifugal force can not effect on the weight 30 and accordingly the actuation hook 34 is made free. In this case, the actuation pin 33 receiving resilience from the spring 37 causes the flange 35 to shift the actuation hook 34 to a non-actuated state to axially move the actuation pin 33 externally from the frame B. When the actuation pin 33 axially moves externally from the frame B, the transfer member 38 and the pivotal lever 39 are also released from press contacting with the actuation member 40. Therefore, the lifting rod 42 is disengaged from the engaging hook 40a of the actuation member 40 by the weights of the movable frame C itself and the supply tray 60, the applicator roller 61, the supply roller 62 and the eccentric gear 57 attached to the movable frame C. As a result, the free end of the movable frame C falls down about the shaft 59 to allow the shorter side of the eccentric gear 57 to mate with the cylindrical gear 55. When the free end of the movable frame C falls down, the applicator roller 61 is released from contacting with the rotary belt A and restored to the original state.

As shown above, the rotary belt sterilizer operates one cycle completely. In some cases, however, the rotary belt A may be halted to run during the operation. For example, even though the cylindrical gear 55 on the deceleration rotary shaft 54 mates with the eccentric gear 57 on the lifting rod 42, the lifting rod 42 during elevation may not have been engaged with the engaging hook 40a on the actuation member 40. In this condition, if the drive rollers 21, 22 stop rotating, the lifting rod 42 stops during elevation. At the same time, the stopped drive roller 21 allows the spring 37 to restore the actuation pin 33 in the flywheel 29 resiliently to the original state to release the pressure that is applied from the pivotal lever 39 to the actuation member 40. Therefore, the movable frame C intends to fall down but the fall is prevented because the cylindrical gear 55 mates with the eccentric gear 57. When the rotary belt A is resumed to run, the engaging hook 40a on the actuation member 40 pivots toward the lifting rod 42 like the above case. At the same time, the eccentric gear 57 is also resumed to rotate from the rotation-halted state. Accordingly, when the numbers of rotations of the cylindrical gear 55 and the eccentric gear 57 before halted to rotate and after resumed to rotate reach predetermined values, or certain periods of time elapse, the sterilizing solution-a is supplied for restoration, like the above case. When the rotary belt A rotates inversely, the above rotary shafts and rotary members operate similarly but rotate in reverse directions opposite to the above.

The above embodiment is shown for the device configured and operative as above but is not limited in the above configuration. For example, known deceleration means such as variable pulleys and variable deceleration gears may be employed to appropriately adjust the interval for applying the sterilizing solution. In addition, the supply amount and frequency of the sterilizing solution may be varied in accordance with a variation in the capacity of the supply pump. Thus, variations and selections can be performed appropriately within the scope of the invention.

According to the present invention with the above configuration, it is possible to separate an applicator roller for the sterilizing solution from a sterilized rotary belt during halts on running of the rotary belt and contact the applicator roller with the rotary belt during running of the rotary belt. This is effective to reduce distortions and abrasions of the rotary belt and the applicator roller in addition to stains attached on the rotary belt as low as possible. Further, only a sufficient amount of the sterilizing solution can be supplied to the rotary belt to eliminate useless consumption of the sterilizing solution. A timer function can be provided in the device itself to supply the sterilizing solution periodically and automatically to save costs for maintenance and management of the device.

## Claims

1. A rotary belt sterilizer comprising:
a drive roller (21, 22) arranged to rotate in synchronization with running of a sterilized rotary belt (A);
an applicator roller (61) for a sterilizing solution (a) configured to apply the sterilizing solution to said sterilized rotary belt (A);
a switching device (42, 59, C) for the applicator roller (61) arranged to contact said applicator roller (61) with said sterilized rotary belt (A) during rotations of said drive roller (21, 22) and separate said applicator roller (61) from said sterilized rotary belt (A) during halts of said drive rotter (21, 22);
a supply tray (60) for the sterilizing solution (a) arranged to supply said sterilizing solution to said applicator roller (61); and
a sterilizing solution supplier arranged to supply said sterilizing solution (a) to said supply tray (60) from a storage tank (65) for the sterilizing solution per predetermined rotations of said drive roller (21, 22).

2. The rotary belt sterilizer according to claim 1, **characterised in that** said drive roller (21, 22), said applicator roller (6), said switching device (42, 59, C), said supply tray (60) and said sterilizing solution supplier are provided in a casing.

3. The rotary belt sterilizer according to claim 1 or 2, **characterised in that** said drive roller (21, 22) is one of a pair of left and right drive rollers (21, 22) normally contacted with said rotary belt (A).

4. The rotary belt sterilizer according to any preceding claim,
**characterised in that** said switching device includes:
a gear (55) provided on a deceleration rotary shaft (54) arranged to rotate/halt in response to rotations/halts of one of said drive rollers (21, 22);
an eccentric gear (57) arranged to detachably mate with said gear (55);
a lifting rod (42) for the applicator roller (61) arranged to lift said applicator roller (61) up and down in response to rotations/halts of said eccentric gear (57);
a flywheel (29) arranged to rotate/halt in response to rotations/halts of the other of said drive rollers (21, 22); and
an engaging unit (C) for the lifting rod (42) arranged to engage with/disengage from said lifting rod (42) in response to rotations/halts of said flywheel (29) when said applicator roller (61) is lifted up.

5. The rotary belt sterilizer according to claim 4 **characterised in that** said engaging unit includes:
a weight (30) movable toward the perimeter of the flywheel (29) in response to a centrifugal force caused by rotations of said flywheel (29);
an actuation pin (33) arranged axially movable on the axial center of a rotary shaft (28) of said flywheel;
an axial movement converter mechanism configured to move said actuation pin (33) axially in response to movement of said weight (30) toward the perimeter of the flywheel (29);
a vertical movement converter mechanism (39) configured to convert axial movement of said actuation pin into vertical movement; and
an engagement hook (40a) configured to complete preparation of engagement with said lifting rod (42) when said vertical movement converter mechanism provides down pressure and disengage from said lifting rod (42) when said vertical movement converter mechanism releases pressure.

6. The rotary belt sterilizer according to claim 5, **characterised in that** said vertical movement converter mechanism includes:
a pivotal lever (39) having a lever end and configured to pivot along the axis of said flywheel (29) in response to movement of said weight (30 toward the perimeter to press said lever (39) end against an end of said actuation rod; and
a spring means configured to normally spring said actuation rod to release pressure applied on said engagement hook (40a).

7. The rotary belt sterilizer according to claim 4,
**characterised in that** said sterilizing solution supplier includes:
a deceleration mechanism containing a worm gear (56) provided on said deceleration rotary shaft (54) and a worm wheel (72) having a flat cam (73) mated with said worm (56);
a supply pump (64) for the sterilizing solution being actuated from a guide pin (75) that impinges on said flat cum (73) in said deceleration mechanism; and
said storage tank (65) for the sterilizing solution arranged in communication with a supply pump (64).

8. The rotary belt sterilizer according to any preceding claim,
**characterised in that** said applicator roller (61) contacts a supply roller (62) for the sterilizing solution that is partly immersed into said sterilizing solution (a) in said supply tray (60) and arranged rotatable therein.

9. The rotary belt sterilizer according to claim 8, **characterised in that** a throttle roller (63) is provided with said supply roller (62).

10. The rotary belt sterilizer according to claim 7, 8 or 9
**characterised in that** said supply pump (64) has an inlet (68) and an outlet (69) each provided with a unidirectional valve in a flow direction of said sterilizing solution (a).

11. The rotary belt sterilizer according to any of claims 7 to 10 when dependent to claim 2,
**characterised in that** said storage tank (65) is detachably attached to said casing, and wherein said storage tank (65) and said casing each have a unidirectional valve at an aperture thereof for attachment to another, said unidirectional valve being opened on attachment and closed on detachment.

## Patentansprüche

1. Rotationsriemensterilisator, der Folgendes umfasst:
eine Antriebsrolle (21, 22), die so gestaltet ist, dass sie synchron mit dem Lauf eines sterilisierten Rotationsriemens (A) rotiert;
eine Applikatorrolle (61) für eine Sterilisationslösung (a), die so konfiguriert ist, dass sie die Sterilisationslösung auf den genannten sterilisierten Rotationsriemen (A) aufbringt;
eine Umschaltvorrichtung (42, 59, C) für die Applikatorrolle (61) mit der Aufgabe, die genannte Applikatorrolle (61) mit dem genannten sterilisierten Rotationsriemen (A) bei Rotationen der genannten Antriebsrolle (21, 22) in Kontakt zu bringen und die genannte Applikatorrolle (61) von dem genannten sterilisierten Rotationsriemen (A) bei Stillstand der genannten Antriebsrolle (21, 22) zu trennen;
eine Aufnahmewanne (60) für die Sterilisationslösung (a) mit der Aufgabe, die genannte Sterilisationslösung auf die genannte Applikatorrolle (61) aufzutragen; und
eine Sterilisationslösungsversorgung mit der Aufgabe, die genannte Sterilisationslösung (a) der Aufnahmewanne (60) von einem Vorratstank (65) für die Sterilisationslösung pro vorbestimmten Rotationen der genannten Antriebsrolle (21, 22) zuzuführen.

2. Rotationsriemensterilisator nach Anspruch 1, **dadurch gekennzeichnet, dass** die genannte Antriebsrolle (21, 22), die genannte Applikatorrolle (6), die genannte Umschaltvorrichtung (42, 59, C), die genannte Aufnahmewanne (60) und die genannte Sterilisationslösungsversorgung in einem Gehäuse vorgesehen sind.

3. Rotationsriemensterilisator nach Anspruch 1 oder 2, **dadurch gekennzeichnet, dass** die genannte Antriebsrolle (21, 22) eine aus einem Paar aus einer linken und einer rechten Antriebsrolle (21, 22) ist, die normalerweise mit dem genannten Rotationsriemen (A) in Kontakt kommen.

4. Rotationsriemensterilisator nach einem der vorherigen Ansprüche, **dadurch gekennzeichnet, dass** die genannte Umschaltvorrichtung Folgendes umfasst:
ein Zahnrad (55), das auf einer Verzögerungsdrehwelle (54) vorgesehen ist, deren Aufgabe es ist, als Reaktion auf eine(n) Rotation/Stillstand von einer der genannten Antriebsrollen (21, 22) zu rotieren/zu halten;
ein Exzenterrad (57) mit der Aufgabe, mit dem genannten Zahnrad (55) ausrückbar zu kämmen;
eine Hubstange (42) für die Applikatorrolle (61) mit der Aufgabe, die genannte Applikatorrolle (61) als Reaktion auf eine(n) Rotation/Stillstand des genannten Exzenterrades (57) zu heben und zu senken;
ein Schwungrad (29) mit der Aufgabe, als Reaktion auf eine(n) Rotation/Stillstand der anderen der genannten Antriebsrollen (21, 22) zu rotieren/zu halten; und
eine Einrückeinheit (C) für die Hubstange (42) mit der Aufgabe, als Reaktion auf eine(n) Rotation/Stillstand des genannten Schwungrades (29) in die genannte Hubstange (42) ein- und aus ihr auszurücken, wenn die genannte Applikatorrolle (61) angehoben wird.

5. Rotationsriemensterilisator nach Anspruch 4, **dadurch gekennzeichnet, dass** die genannte Einrückeinheit Folgendes umfasst:
ein Gewicht (30), das sich als Reaktion auf eine durch Rotationen des genannten Schwungrades (29) erzeugte Zentrifugalkraft in Richtung auf den Umfang des Schwungrades (29) bewegen kann;
einen Betätigungsbolzen (33), der so angeordnet ist, dass er axial auf der axialen Mitte einer Drehwelle (28) des genannten Schwungrades beweglich ist;
einen Axialbewegungswandlermechanismus, der so konfiguriert ist, dass er den genannten Betätigungsbolzen (33) als Reaktion auf eine Bewegung des genannten Gewichtes (30) axial in Richtung auf den Umfang des genannten Schwungrades (29) bewegt;
einen Senkrechtbewegungswandlermechanismus (39), der so konfiguriert ist, dass er eine axiale Bewegung des genannten Betätigungsbolzens in eine Senkrechtbewegung umwandelt; und
einen Einrückhaken (40a), der so konfiguriert ist, dass er die Vorbereitung des Einrückens in die genannte Hubstange (42) vervollständigt, wenn der genannte Senkrechtbewegungswandlermechanismus einen Abwärtsdruck erzeugt und von der genannten Hubstange (42) ausrückt, wenn der genannte Senkrechtbewegungswandlermechanismus den Druck löst.

6. Rotationsriemensterilisator nach Anspruch 5, **dadurch gekennzeichnet, dass** der genannte Senkrechtbewegungswandlermechanismus Folgendes umfasst:
einen Schwenkhebel (39) mit einem Hebelende, der so konfiguriert ist, dass er entlang der Achse des genannten Schwungrades (29) als Reaktion auf eine Bewegung des genannten Gewichts (30) in Richtung auf den Umfang schwenkt, um das genannte Ende des Hebels (39) gegen ein Ende der genannten Betätigungsstange zu drücken; und
eine Feder, die so konfiguriert ist, dass sie normalerweise die genannte Betätigungsstange federt, um den auf den genannten Einrückhaken (40a) wirkenden Druck zu lösen.

7. Rotationsriemensterilisator nach Anspruch 4, **dadurch gekennzeichnet, dass** die genannte Sterilisationslösungsversorgung Folgendes umfasst:
einen Verzögerungsmechanismus, der ein Schneckenrad (56) aufweist, das auf der genannten Verzögerungsdrehwelle (54) vorgesehen ist, und ein Schneckenrad (72) mit einer Plankurvenscheibe (73), die mit dem genannten Schneckenrad (56) zusammengesteckt wird;
eine Versorgungspumpe (64) für die Sterilisationslösung, die von einem Führungsbolzen (75) betätigt wird, der auf die genannte Plankurvenscheibe (73) in dem genannten Verzögerungsmechanismus auftrifft; und
den genannten Vorratstank (65) für die Sterilisationslösung, der in Verbindung mit einer Versorgungspumpe (64) angeordnet ist.

8. Rotationsriemensterilisator nach einem der vorherigen Ansprüche, **dadurch gekennzeichnet, dass** die genannte Applikationsrolle (61) mit einer Zuführungsrolle (62) für die Sterilisationslösung in Kontakt kommt, die teilweise in die genannte Sterilisationslösung (a) in der genannten Aufnahmewanne (60) eingetaucht und drehbar darin angeordnet ist.

9. Rotationsriemensterilisator nach Anspruch 8, **dadurch gekennzeichnet, dass** eine Drosselrolle (63) mit der genannten Zuführungsrolle (62) vorgesehen ist.

10. Rotationsriemensterilisator nach Anspruch 7, 8 oder 9, **dadurch gekennzeichnet, dass** die genannte Versorgungspumpe (64) einen Einlass (68) und einen Auslass (69) hat, die jeweils mit einem Rückschlagventil in einer Strömungsrichtung der genannten Sterilisationslösung
(a) vorgesehen ist.

11. Rotationsriemensterilisator nach einem der Ansprüche 7 bis 10 in Abhängigkeit von Anspruch 2, **dadurch gekennzeichnet, dass** der genannte Vorratstank (65) lösbar an dem genannten Gehäuse montiert ist, und wobei der genannte Vorratstank (65) und das genannte Gehäuse an einer Öffnung darin zum Anbringen aneinander jeweils ein Rückschlagventil aufweisen, wobei das genannte Rückschlagventil beim Anbringen geöffnet und beim Abnehmen geschlossen wird.

## Revendications

1. Un stérilisateur à courroie rotative, comprenant :
un rouleau d'entraînement (21, 22) agencé pour tourner en synchronisme avec la course d'une courroie rotative stérilisée (A) ;
un cylindre applicateur (61) pour une solution stérilisante (a), configuré pour appliquer la solution stérilisante à ladite courroie rotative stérilisée (A) ;
un dispositif de commutation (42, 59, C) pour le cylindre applicateur (61) agencé pour mettre en contact ledit cylindre applicateur (61) avec ladite courroie rotative stérilisée (A) pendant les rotations dudit rouleau d'entraînement (21, 22) et séparer ledit cylindre applicateur (61) de ladite courroie rotative stérilisée (A) pendant les arrêts dudit rouleau d'entraînement (21, 22) ;
un plateau d'alimentation (60) en solution stérilisante (a), agencé pour fournir ladite solution stérilisante audit cylindre applicateur (61) ; et
un dispositif d'alimentation en solution stérilisante agencé pour fournir ladite solution stérilisante (a) audit plateau d'alimentation (60) depuis un réservoir de stockage (65) de solution stérilisante en fonction de rotations prédéterminées dudit rouleau d'entraînement (21, 22).

2. Le stérilisateur à courroie rotative selon la revendication 1, **caractérisé en ce que** ledit rouleau d'entraînement (21, 22), ledit cylindre applicateur (61), ledit dispositif de commutation (42, 59, C), ledit plateau d'alimentation (60) et ledit dispositif d'alimentation en solution stérilisante sont placés dans un carter.

3. Le stérilisateur à courroie rotative selon la revendication 1 ou 2, **caractérisé en ce que** ledit rouleau d'entraînement (21, 22) est l'un d'une paire de rouleaux d'entraînement gauche et droit (21, 22), normalement en contact avec ladite courroie rotative (A).

4. Le stérilisateur à courroie rotative selon l'une quelconque des revendications précédentes, **caractérisé en ce que** ledit dispositif de commutation comprend :
une roue dentée (55) prévue sur un arbre rotatif à décélération (54), agencé pour tourner et s'arrêter en réponse aux rotations et aux arrêts de l'un desdits rouleaux d'entraînement (21, 22) ;
une roue à excentrique (57) agencée pour s'adapter de façon détachable avec ladite roue dentée (55) ;
une bielle de levage (42) pour le cylindre applicateur (61), qui relève et abaisse le cylindre applicateur (61) en réponse aux rotations et aux arrêts de ladite roue à excentrique (57) ;
un volant (29) agencé pour tourner et s'arrêter en réponse aux rotations et aux arrêts de l'autre rouleau de la paire de rouleaux d'entraînement (21, 22) ; et
une unité d'accouplement (C) pour la bielle de levage (42) agencée pour s'accoupler avec ladite bielle de levage (42) ou s'en découpler en réponse aux rotations et aux arrêts dudit volant (29) lorsque ledit cylindre applicateur (61) est relevé.

5. Le stériliseur à courroie rotative selon la revendication 4, **caractérisé en ce que** ladite unité d'accouplement comprend :
un poids (30) qui peut être déplacé vers le périmètre du volant (29) en réponse à une force centrifuge produite par des rotations dudit volant (29) ;
une goupille de commande (33) agencée pour pouvoir être déplacée axialement sur l'axe d'un arbre rotatif (28) dudit volant ;
un mécanisme de conversion en mouvements axiaux configuré pour déplacer axialement ladite goupille de commande (33) en réponse au mouvement dudit poids (30) vers le périmètre du volant (29) ;
un mécanisme de conversion en mouvements verticaux (39) configuré pour convertir le mouvement axial de ladite goupille de commande en un mouvement vertical ; et
un crochet d'accouplement (40a) configuré de sorte à compléter la préparation de l'accouplement avec ladite bielle de levage (42) lorsque ledit mécanisme de conversion en mouvements verticaux fournit une pression vers le bas et à se découpler de ladite bielle de levage (42) lorsque ledit mécanisme de conversion en mouvements verticaux relâche la pression.

6. Le stérilisateur à courroie rotative selon la revendication 5, **caractérisé en ce que** le mécanisme de conversion des mouvements verticaux comprend :
un levier à pivot (39) ayant une extrémité de levier, et configuré pour pivoter le long de l'axe dudit volant (29) en réponse au mouvement dudit poids (30) vers le périmètre pour presser ladite extrémité de levier (39) contre une extrémité de ladite bielle de commande ; et
un moyen formant ressort configuré pour rappeler normalement ladite bielle de commande afin de relâcher la pression appliquée sur ledit crochet d'accouplement (40a).

7. Le stérilisateur à courroie rotative selon la revendication 4, **caractérisé en ce que** ledit alimentateur en solution stérilisatrice comprend :
un mécanisme de décélération contenant une vis sans fin (56) prévue sur ledit arbre de décélération rotatif (54) et une roue tangente (72) ayant un plateau courbe lisse à fente (73) qui s'adapte avec ladite vis sans fin (56) ;
une pompe d'alimentation (64) en solution stérilisante, commandée à partir d'une goupille de guidage (75) qui pénètre dans la fente du plateau courbe lisse (73) dans ledit mécanisme de décélération ; et
ledit réservoir de stockage (65) de solution stérilisante, agencé pour communiquer avec une pompe d'alimentation (64).

8. Le stérilisateur à courroie rotative selon l'une quelconque des revendications précédentes, **caractérisé en ce que** ledit cylindre applicateur (61) entre en contact avec un cylindre alimentateur (62) en solution stérilisante, ce cylindre (62) étant partiellement immergé dans ladite solution stérilisante (a) dans ledit plateau d'alimentation (60) et est agencé pour pouvoir tourner dans cette solution.

9. Le stérilisateur à courroie rotative selon la revendication 8, **caractérisé en ce qu'**un rouleau régulateur (63) est prévu avec ledit cylindre alimentateur (62).

10. Le stérilisateur à courroie rotative selon la revendication 7, 8 ou 9, **caractérisé en ce que** ladite pompe d'alimentation (64) a un orifice d'admission (68) et un orifice de refoulement (69) dont chacun est pourvu d'une soupape unidirectionnelle dans la direction de débit de ladite solution stérilisante (a).

11. Le stérilisateur à courroie rotative selon l'une quelconque des revendications 7 à 10 lorsque dépendantes de la revendication 2, **caractérisé en ce que** ledit réservoir de stockage (65) est attaché de façon amovible sur ledit carter, et **en ce que** ledit réservoir de stockage (65) et ledit carter sont tous deux pourvus d'une soupape unidirectionnelle au niveau de l'un de leurs orifices pour leur fixation l'un à l'autre, ladite soupape unidirectionnelle étant ouverte quand le réservoir et le carter sont réunis, et fermée lorsqu'ils sont séparés.
